**Europäisches Patentamt**

**European Patent Office**

**Office européen des brevets**

(19)

(11) Veröffentlichungsnummer : **0 054 675**
**B1**

(12) # EUROPÄISCHE PATENTSCHRIFT

(45) Veröffentlichungstag der Patentschrift :
**15.05.85**

(51) Int. Cl.⁴ : **G 01 N 33/53, G 01 N 33/543**

(21) Anmeldenummer : **81108754.3**

(22) Anmeldetag : **22.10.81**

(54) **Verfahren zur Enzymimmunobestimmung in heterogener Phase.**

(30) Priorität : **23.12.80 DE 3048884**

(43) Veröffentlichungstag der Anmeldung :
**30.06.82 Patentblatt 82/26**

(45) Bekanntmachung des Hinweises auf die Patenterteilung : **15.05.85 Patentblatt 85/20**

(84) Benannte Vertragsstaaten :
**AT BE CH DE FR GB IT LI LU NL SE**

(56) Entgegenhaltungen :
**DE-A- 2 938 646**
**FR-A- 2 365 124**
**FR-A- 2 418 463**
**US-A- 3 839 153**
**Centrifugal analysers in clinical chemistry, 1980,**
**Praeger Publishers, Seiten 3-13, 125-131.**
**J. Clin. chem. Clin. Biochem. 18, 1980, Seiten 197-**
**208.**

(73) Patentinhaber : **BOEHRINGER MANNHEIM GMBH**
**Patentabteilung, Abt. E Sandhofer Strasse 112-132**
**Postfach 31 01 20**
**D-6800 Mannheim 31 Waldhof (DE)**

(72) Erfinder : **Evanega, George R., Dr.**
**3705 Carmel Drive**
**Carmel, IN 46032 (US)**
Erfinder : **Albert, Winfried, Dr.**
**Moosstrasse 10**
**D-8121 Pähl (DE)**

(74) Vertreter : **Weickmann, Heinrich, Dipl.-Ing. et al**
**Patentanwälte Dipl.-Ing. H.Weickmann Dipl.-Phys.Dr.**
**K.Fincke Dipl.-Ing. F.A.Weickmann Dipl.-Chem. B.**
**Huber Dr.-Ing. H. Liska Dipl.-Phys.Dr. J. Prechtel**
**Postfach 860820**
**D-8000 München 86 (DE)**

## Beschreibung

Die Erfindung betrifft ein Verfahren zur Bestimmung einer Komponente der Reaktion zwischen einem spezifisch bindenden Protein und einer damit bindefähigen Substanz unter Anwendung des Prinzips des Enzymimmuno-Verfahrens in heterogener Phase (ELISA).

Aufgrund der ständig steigenden Zahl von klinisch-chemischen Analysen von Serumbestandteilen und ähnlichen biologischen Materialien gewinnt die Automatisierbarkeit derartiger Methoden ständig weiter an Bedeutung. Nur durch Automatisierung läßt sich die stark steigende Zahl derartiger Bestimmungen bewältigen und dabei auch der erforderliche Arbeitsaufwand pro Bestimmung herabsetzen. In neuerer Zeit haben dabei Analysenautomaten, die auf dem Zentrifugalanalysenprinzip beruhen, große Bedeutung erlangt, da sie eine weitere Erhöhung der Analysenfrequenz ohne besondere Steigerung der Anlagenkosten ermöglicht (G-i-T, Fachz. Lab. 21 (1977), 866-874).

Für die klinische Diagnostik steigt in neuerer Zeit auch die Bedeutung von Verfahren zur Bestimmung von Partnern von Immunreaktionen. Einen wesentlichen Fortschritt brachte dabei die Methode der Radioimmunanalyse (RIA), da sie eine wesentliche Erhöhung der Empfindlichkeit derartiger Bestimmungen mit sich brachte. Ein Nachteil des RIA, nämlich die Notwendigkeit, mit radioaktiven Substanzen umzugehen, wurde beseitigt durch den Ersatz der radioaktiven Markierung durch eine Enzymmarkierung, was zum sogenannten Enzym-Immuno-Assay (EIA) führte. Bei einer Ausführungsform eines derartigen Verfahren, die üblicherweise als ELISA-Methode bezeichnet wird, wird eine Komponente der Reaktion zwischen einem spezifisch bindenden Protein und einer damit bindefähigen Substanz bestimmt, indem man eine Probelösung mit unbekanntem Gehalt an der zu bestimmenden Substanz mit einer vorher festgelegten Menge einer Bindungskomponente in unlöslicher Form in Gegenwart eines Reagenz inkubiert, welche eine bekannte Menge einer der Bindungskomponenten in enzymmarkierter Form enthält. Nach Trennung der flüssigen von der festen Phase stellt die in der flüssigen Phase verbliebene Aktivität des Markierungsenzyms ein Maß für die in der Probelösung vorhandene unbekannte Menge der zu bestimmenden Komponente der Immunreaktion dar (vgl. J. Clin. Chem. Clin. Biochem. Vol. 18 (1980) 197-208). Dieser heterogene Enzymimmunotest beseitigt zwar die Notwendigkeit, mit radioaktiven Substanzen zu arbeiten, der dabei erforderliche Trennungsschritt bereitet jedoch bei der Automatisierung Schwierigkeiten und erfordert zusätzliche Maßnahmen, wie sie beispielsweise in der DE-OS 27 36 527 beschrieben werden.

Weiter bekannt ist auch die sogenannte EMIT-Technik, d. h. ein Enzymimmunoassay in homogener Phase. Aus dem Buch Centrifugal Analysers in Clinical Chemistry, 1980, Praeger Publishers, edited by P. Price and K. Spencer, Seiten 3 bis 11 und 125 bis 131 war es auch schon bekannt, diesen EMIT-Test in einem Zentrifugenrotor durchzuführen. Dies war möglich, da die EMIT-Technik in homogener Phase durchgeführt wird und deswegen keine Trennung von gebundener und freier Enzymaktivität nötig ist. Nachteil des EMIT-Verfahrens ist jedoch die weitaus geringere Empfindlichkeit.

Der Erfindung liegt nun die Aufgabe zugrunde, ein heterogenes Enzym-Immuno-Testverfahren so zu gestalten, daß die Automatisierbarkeit verbessert wird und eine Durchführung an Zentrifugalanalysatoren ermöglicht wird.

Gelöst wird diese Aufgabe erfindungsgemäß durch ein Verfahren zur Bestimmung einer Komponente der Reaktion zwischen einem spezifisch bindenden Protein und einer damit bindefähigen Substanz nach dem Prinzip des heterogenen Enzymimmunoassay (ELISA), das dadurch gekennzeichnet ist, daß man die Messung der Enzymaktivität im Rotor eines Zentrifugalanalysenautomaten während der Einwirkung der erhöhten Schwerkraft durchführt, während feste und flüssige Phase noch miteinander in Kontakt stehen.

Die Erfindung beruht auf der sehr überraschenden Tatsache, daß unter dem Einfluß der Schwerkraft in einem Zentrifugenrotor der an die feste Phase gebundene Anteil des Enzyms an der Enzymreaktion praktisch nicht teilnimmt, so daß unter den erfindungsgemäßen Bedingungen bei der Messung der Enzymaktivität nur das in der flüssigen Phase vorliegende Enzym wirksam wird, während das damit in direktem Kontakt stehende, infolge der Einwirkung der erhöhten Schwerkraft aber an die Behälterwand gebundene Enzym bei der Bestimmung nicht erfaßt wird. Bei einem Zentrifugalanalysator werden ja die Komponenten einer Reaktion üblicherweise zuerst unter dem Einfluß der Schwerkraft gemischt und dann in ein Bestimmungsgefäß weitergefördert, welches als eine Art Küvette ausgebildet ist, in der während des Rotorlaufs die Messung durchgeführt wird. An der Küvettenwand, auf welche die Schwerkraft wirkt, sammelt sich nun beim Verfahren der Erfindung das an die feste Phase gebundene Markerenzym an, wird also insoweit zwar dem Lichtstrahl, der für die Messung durch die Küvette geschickt wird, entzogen, bleibt aber weiterhin in Kontakt mit der Reagenzlösung und kann daher auch an Umsetzungen in der Reagenzlösung weiter teilnehmen. Diese Teilnahme von in fester Phase gebundenen Enzymen an Reaktionen in der Lösung wird gerade bei den trägergebundenen Enzymen technisch in weitem Umfang ausgenutzt. Falls man überhaupt eine Einwirkung des Schwerkraftfelds bei derartigen Reaktionen erwartet hätte, so allenfalls eine verstärkte Teilnahme des an die Behälterwand zentrifugierten Enzyms, da eine Wanderung der Reaktionspartner unter dem Einfluß der Schwerkraft, wenn überhaupt, so nur in Richtung ihrer Wirkung, d. h. zum in fester Phase vorliegenden Enzym, aber nicht vom Festphasenenzym weg zu erwarten war.

Die Erfindung macht es möglich, die bis jetzt als absolut unumgänglich angesehenen Verfahrensschritte : Trennung von fester und flüssiger Phase und Waschen der festen Phase (Dt. Ges. f. Klin. Chemie, Mitteilungen 1/79, 22-30), überflüssig gemacht werden und das Verfahren auf üblichen Zentrifugenanalysatoren durchführbar wird. Das wesentliche Hindernis bei der Durchführung der ELISA-Technik auf Analysenautomaten wird damit beseitigt.

Die unlösliche Komponente der Enzymreaktion wird beim erfindungsgemäßen Verfahren vorzugsweise in trägergebundener Form eingesetzt. Die Trägerbindung kann nach den für die Immobilisierung von biologisch aktiven Substanzen dem Fachmann bekannten Methoden erfolgen. Alternativ kann die unlösliche Komponente auch in vernetzter Form eingesetzt werden, beispielsweise durch an sich bekannte Umsetzung mit multifunktionalen Vernetzungsmitteln. Derartige Vernetzungsmittel, wie Glutardialdehyd, usw. sind dem Fachmann bekannt und brauchen hier nicht näher erläutert zu werden. Vernetzung und Trägerfixierung können auch kombiniert angewendet werden, indem eine Komponente der Immunreaktion auf einem Träger durch Vernetzung fixiert wird. Besonders zweckmäßig wird die unlösliche Komponente in Teilchenform eingesetzt, entweder an einen teilförmigen Träger fixiert oder durch trägerfreie Vernetzung in Teilchenform überführt.

Der Träger für die unlösliche Komponente kann, wenn er verwendet wird, flüssig oder fest sein. Gemäß einer besonders bevorzugten Ausführungsform der Erfindung setzt man die unlösliche Komponente der Immunreaktion an Latexteilchen gebunden ein. Ein besonderer Vorzug von Latexpartikeln liegt in ihrer Dichte, die sie in wäßriger Lösung in Schwebe hält. Zu diesem Zweck besonders geeignete Latexpartikel sind hydrophile Latexpartikel, die aus einem Homo- oder Copolymerisat von in Wasser schwerlöslichen Monomeren bestehen und durch Emulsionspolymerisation in Anwesenheit eines wasserlöslichen, Radikale bildenden Initiators, jedoch ohne Zusatz eines Emulgators, Stabilisators oder Netzmittels, herstellbar sind. Die Herstellung erfolgt in wäßriger Dispersion des oder der Monomeren durch Emulsionspolymerisation in Abwesenheit von Sauerstoff.

Im Rahmen der Erfindung sind jedoch auch andere Träger geeignet, wie sie üblicherweise für die Fixierung von biologisch aktiven Proteinen verwendet werden, beispielsweise unlösliche Kohlenhydrate, wie Cellulosederivate, vernetztes Dextran, hydrophile Polymere und Copolymere, insbesondere auf Basis von Acrylamid und dergleichen.

Die im Rahmen der Erfindung erforderliche Schwerkraft entspricht den bei handelsüblichen Zentrifugalanalysenautomaten normalerweise auftretenden Werten.

Alle übrigen Bedingungen, die beim Verfahren einzuhalten sind, entsprechen den für die jeweilige Bestimmung nach der ELISA-Technik bekannten Bedingungen. Sie werden im wesentlichen durch die pH- und Puffer-Abhängigkeit des Markerenzyms bestimmt. Diese Bedingungen sind dem Fachmann bekannt. Auch Konzentrationen und Inkubationszeiten können den bisher bekannten Werten entsprechen.

Auch hinsichtlich der verwendbaren Markerenzyme bestehen keine Beschränkungen. Alle für die ELISA-Technik geeigneten Markerenzyme lassen sich auch im Rahmen der Erfindung anwenden.

Das erfindungsgemäße Verfahren eignet sich für die verschiedenen bekannten Abwandlungen der ELISA-Technik, also die sogenannte kompetitive Enzym-Immuno-Testmethode, die kompetitive Sandwich-Methode, die Sandwich-Antigen-Methode, die Sandwich-Antikörper-Methode und die immunoenzymometrische Methode. Diese Methoden sind in der oben erwähnten Literaturstelle J. Clin. Chem. Clin. Biochem. schematisch erläutert. Beispiele für geeignete Markerenzyme sind Peroxidase, Glucoseoxidase. Galactosidase, alkalische Phosphatase, Glucoamylase, Acetylcholinesterase und Katalase.

Typische Beispiele für Antigene oder Haptene, die mit dem erfindungsgemäßen Verfahren bestimmt werden können, sind Thyroxin, Digoxin, Östriol, $\alpha_1$-Fetoprotein, Insulin, Thyrotropin, Ferritin, carcinoembryonales Antigen, $HB_2$-Antigen und ähnliche. In gleicher Weise können die korrespondierenden oder beliebige andere Antikörper bestimmt werden.

Die folgenden Beispiele erläutern die Erfindung weiter.

## Beispiel 1

Verwendete Reagenzien :

a) Sepharose-Anti-$T_4$-Antikörper ($T_4$ = Tetrajod-Tyrosin = Thyroxin)

1 g handelsübliche bromcyanaktivierte vernetzte Agarose (Sephasose) wurde nach Vorschrift des Herstellers mit 10 ml Anti-$T_4$-Immunglobulin (Anti-$T_4$-IgG von Kaninchen) gekuppelt, wobei das Anti-$T_4$-IgG aus Anti-$T_4$-Antiserum durch Fällung mit 14 %-iger (w/v) Ammoniumsulfatlösung gewonnen worden ist (Probelösung)

b) $T_4$-Glucoseoxidase ($T_4$-GOD)

200 mg (= 1,07 $\mu$M) lyophilisierte Glucoseoxidase werden in 20 ml 0,1 M Phosphatpuffer, pH 8,6, gelöst und tropfenweise unter Kühlen und Rühren mit 18,8 ml Dimethylformamid versetzt. Zu dieser Lösung werden 15 mg (= 15,4 $\mu$M) BOC-$T_4$-OSu (Su = Succinimid), in 1,2 ml Dimethylformamid gelöst, innerhalb 3 Stunden in 400 $\mu$l-Portionen zugesetzt. Die Reaktionslösung wird 20 Stunden bei 4 °C unter Rühren und Lichtausschluß gehalten. Eine auftretende leichte Trübung wird abzentrifugiert. Die überstehende Lösung wird an Molekularsieb auf Basis von vernetztem Dextran (Sephasex G-25, fine) in 40 mM Tris-Puffer, pH 7,5, und 150 mM Natriumchlorid aufgetrennt. Die erste Fraktion (Front) wird aufgefangen : 86 ml. Die erhaltene Lösung wird zweimal gegen 12 l 40 mM Phosphatpuffer, pH 6,5, dialysiert. Die so erhaltene GOD-T$_4$ enthaltende Lösung wird für die folgenden Experimente im Verhältnis 1 : 10 mit Phosphatpuffer, pH 6,5, verdünnt.

c) Enzymsubstratlösung

20 mg Peroxidase (POD), 4,6 g 2,2'-Azino-di-[3-äthylbenzthiazolinsulfonat (6)] und 50 g Glucose werden mit 0,01 M Phosphat/Citrat-Puffer, pH 5,6, und 0,1 % Polyoxyäthylensorbitanmonooleat (Tween 20) auf ein Endvolumen von 1 l gebracht.

d) Versuchsdurchführung

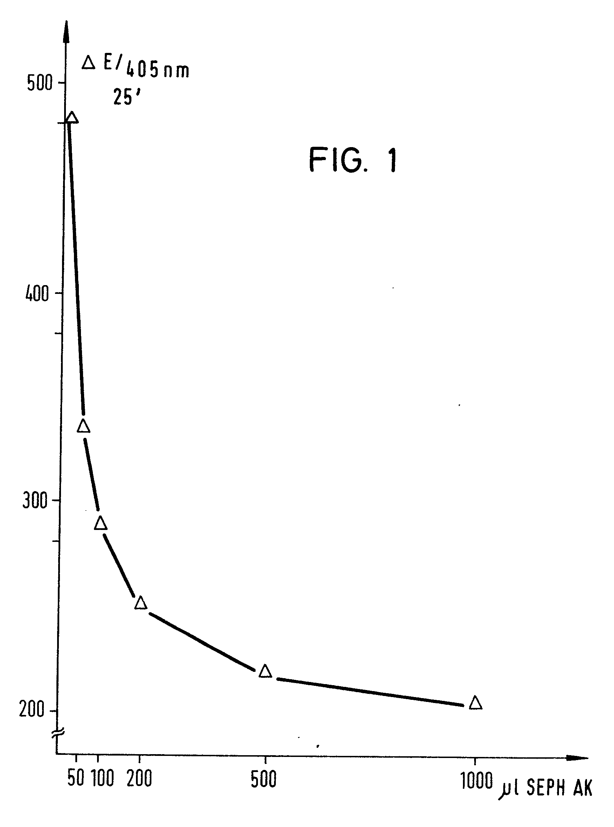

Verschiedene, jeweils bekannte Menge an der unter a) beschriebenen Sepharose-Anti-T$_4$-IgG-Lösung (50/100/200/500/1 000 µl) werden mit jeweils 100 µl der unter b) beschriebenen T$_4$-GOD-Lösung vermischt und 15 Minuten bei Raumtemperatur gerührt. 35 µl dieses Inkubationsgemisches werden in die Probemulde eines handelsüblichen Zentrifugalanalysenautomaten (CentrifiChem 400) gegeben. In den Reagenzbereich werden 60 µl des unter c) beschriebenen Enzymsubstrat-Gemisches gefüllt. Danach wird zentrifugiert und währenddessen die Extinktion bei 405 nm gemessen. Fig. 1 der Zeichnung zeigt in graphischer Darstellung die Abnahme der Extinktion, gemessen nach 25 Minuten ab Beginn der Zentrifugation, gegen die Zunahme des Sepharose-Anti-T$_4$-IgG-Gehalts aufgetragen.

Beispiel 2

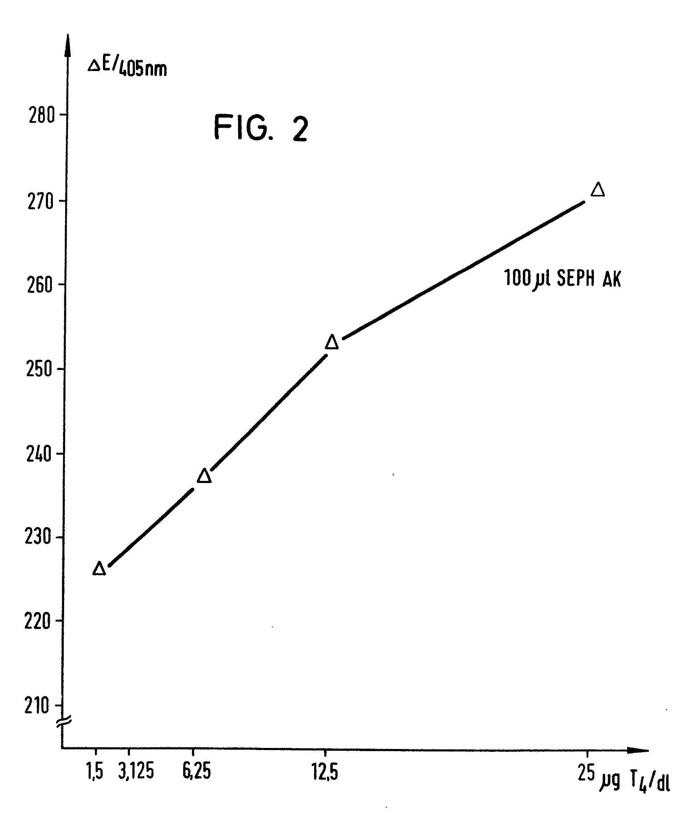

100 µl Sepharose-Anti-T$_4$-IgG, 9-0 µl 0,1 M Phosphatpuffer, pH 7,0, 100 µl T$_4$-GOD-Lösung und 100 µl eines T$_4$-Standards in 0,1 M Phosphatpuffer, pH 7,0, werden 1 Stunde gerührt. 35 µl dieses Inkubationsgemisches werden wie in Beispiel 1 d) beschrieben am Zentrifugalanalysenautomaten weiterbehandelt. Durch Einsatz von T$_4$-Standardlösungen unterschiedlichen T$_4$-Gehalts wird eine Eichkurve erstellt. Die ermittelten Werte sind in Fig. 2 dargestellt. Anhand dieser Eichkurve lassen sich unbekannte T$_4$-Konzentrationen bestimmen. Hierzu wird in der oben beschriebenen Weise verfahren, jedoch wird anstelle der T$_4$-Standardlösung eine entsprechende Menge der Probe mit unbekanntem T$_4$-Gehalt eingesetzt.

Beispiel 3

Verwendete Reagenzien

a) Latex-Anti-T$_4$-Antikörper

T$_4$-Antikörper aus einem Anti-T$_4$-Serum vom Schaf werden an Homopolyglycidylmethacrylat-Latexpartikel kovalent gebunden.

b) T$_4$-β-Galactosidase (T$_4$-β-Gal)

T$_4$ wird analog Beispiel 1 b) mit β-Gal markiert.

c) β-Gal-Substratlösung

450 mg/l p-Nitrophenyl-β-galactosidase (Fa. Sigma)
100 mM Natriumchlorid
10 mM Magnesiumchlorid
10 mM Tris-Puffer/HCl, pH-Wert 7,3
0,4 % (V/) Mercaptoäthanol

d) Versuchsdurchführung

0,5 ml einer verdünnten Latex-Anti-T$_4$-Suspension werden mit 100 µl einer T$_4$-Serum-Standardlösung mit verschiedenem, jeweils bekanntem T$_4$-Behalt und 100 µl einer T$_4$-β-Gal-Konjugatlösung versetzt. Das Reaktionsgemisch wird 30 Minuten bei Raumtemperatur stehen gelassen. 35 µl des jeweiligen Inkubationsgemisches werden in die Probemulde eines handelsüblichen Zentrifugalanalysenautomaten (CentrifiChem 400) gegeben. In den Reagenzbereich dieses Zentrifugalanalysators werden 60 µl der unter c) beschriebenen Substratlösung gefüllt. Danach wird zentrifugiert und die Extinktion bei einer Wellenlänge von 405 nm gemessen.

Nachdem auf diese Weise die T$_4$-Eichkurve ermittelt wurde, wurden anschließend in derselben Weise Bestimmungen durchgeführt, bei denen anstelle von 100 µl T$_4$-Standardserum jeweils 100 µl von Proben mit unbekanntem T$_4$-Gehalt eingesetzt wurden.

**Patentansprüche**

1. Verfahren zur Bestimmung einer Komponente der Reaktion zwischen einem spezifisch bindenden

0 054 675

Protein und einer damit bindefähigen Substanz nach dem Prinzip des heterogenen Enzymimmunoassay (ELISA), dadurch gekennzeichnet, daß man die Messung der Enzymaktivität im Rotor eines Zentrifugalanalysenautomaten während der Einwirkung der erhöhten Schwerkraft durchführt, während feste und flüssige Phase noch miteinander in Kontakt stehen.

2. Verfahren nach Anspruch 1, dadurch gekennzeichnet, daß man die unlösliche Komponente in trägergebundener Form einsetzt.

3. Verfahren nach Anspruch 1 oder 2, dadurch gekennzeichnet, daß man die unlösliche Komponente in vernetzter Form einsetzt.

4. Verfahren nach einem der Ansprüche 1 bis 3, dadurch gekennzeichnet, daß man die unlösliche Komponente in Teilchenform einsetzt.

5. Verfahren nach Anspruch 4, dadurch gekennzeichnet, daß man die unlösliche Komponente an Latexteilchen gebunden einsetzt.

6. Verfahren nach einem der vorhergehenden Ansprüche, dadurch gekennzeichnet, daß man als unlösliche Komponente einen Antikörper oder Anti-Antikörper verwendet.

7. Verfahren nach Anspruch 6, dadurch gekennzeichnet, daß man als enzymmarkierte Komponente ein Antigen, Hapten, einen Antikörper oder einen Anti-Antikörper verwendet.

8. Verfahren nach einem der Ansprüche 1 bis 6, dadurch gekennzeichnet, daß man als unlösliche Komponente ein Antigen oder Hapten und als enzymmarkierte Komponente einen Antikörper verwendet.


**Claims**

1. Process for the determination of a component of the reaction between a specific binding protein and a substance bindable therewith according to the principle of heterogenous enzyme immunoassay (ELISA), characterised in that one carries out the measurement of the enzyme activity in the rotor of a centrifugal automatic analyser during the action of the increased gravitational force while solid and liquid phase are still in contact with one another.

2. Process according to claim 1, characterised in that one uses the insoluble component in carrier-bound form.

3. Process according to claim 1 or 2, characterised in that one uses the insoluble component in cross-linked form.

4. Process according to one of claims 1 to 3, characterised in that one uses the insoluble component in particulate form.

5. Process according to claim 4, characterised in that one uses the insoluble component bound to latex particles.

6. Process according to one of the preceding claims, characterised in that one uses an antibody or anti-antibody as insoluble component.

7. Process according to claim 6, characterised in that one uses an antigen, a hapten, an antibody or an anti-antibody as enzyme-marked component.

8. Process according to one of claims 1 to 6, characterised in that one uses an antigen or a hapten as insoluble component and an antibody as enzyme-marked component.


**Revendications**

1. Procédé de détermination d'un constituant de la réaction entre une protéine liant spécifiquement et une substance capable de se lier à celle-ci suivant le principe du dosage immunoenzymatique hétérogène (ELISA), caractérisé en ce qu'on effectue la mesure de l'activité enzymatique dans le rotor d'un appareil automatique d'analyse centrifuge pendant l'action de la gravité augmentée, tandis que les phases solide et liquide sont encore en contact entre elles.

2. Procédé suivant la revendication 1, caractérisé en ce qu'on utilise le constituant insoluble sous forme liée à un support.

3. Procédé suivant la revendication 1 ou 2, caractérisé en ce qu'on utilise le constituant insoluble sous forme réticulée.

4. Procédé suivant l'une des revendications 1 à 3, caractérisé en ce qu'on utilise le constituant insoluble sous forme de particules.

5. Procédé suivant la revendication 4, caractérisé en ce qu'on utilise le constituant insoluble lié à des particules de latex.

6. Procédé suivant l'une des revendications précédentes, caractérisé en ce qu'on utilise comme constituant insoluble un anticorps ou un anti-anticorps.

7. Procédé suivant la revendication 6, caractérisé en ce qu'on utilise comme constituant marqué par une enzyme un antigène, un haptène, un anticorps ou un anti-anticorps.

8. Procédé suivant l'une des revendications 1 à 6, caractérisé en ce qu'on utilise comme constituant insoluble un antigène ou un haptène et comme constituant marqué par une enzyme, un anticorps.

5

FIG. 1

FIG. 2

$\triangle$E/405nm

100 µl SEPH AK

1,5  3,125  6,25  12,5  25 µg T$_4$/dl